# EUROPEAN PATENT APPLICATION

(11) **EP 4 468 306 A1**
(43) Date of publication of application: **27.11.2024**
(21) Application number: 23174528.2
(22) Date of filing: 22.05.2023
(51) Int. Cl.: G16H 30/40, G16H 40/60, G16H 50/00

(54) **COMPUTER-IMPLEMENTED METHOD FOR PROVIDING A DOSE REDUCED CT PROTOCOL FOR A FOLLOW-UP STUDY OF A PATIENT**

(71) Applicant: Siemens Healthineers AG, 91301 Forchheim (DE)
(72) Inventor: Sundarapandian, Manivannan, 560030 Bangalore (IN); Seemanapalli, Supriya, 560069 Bangalore (IN); Raj, Ayush, 560037 Bengaluru (IN)
(74) Representative: Siemens Healthineers Patent Attorneys

(57) **Abstract**

Computer-implemented method for providing a dose reduced CT protocol for a follow-up study of a patient, comprising:
- receiving (100) patient information (D) of a primary study, including at least one primary CT protocol and/or at least one primary medical image of an examination area;
- receiving (200) a follow-up information (Df) comprising at least one initial follow-up CT protocol;
- receiving (300) a plurality of dose reduction factors (dᵢ), wherein the dose reduction factors (dᵢ) define a dose reduction for a follow-up study with a CT protocol (Pᵢ) compared to a follow-up study with the primary CT protocol;
- generating (400) at least one preview image Im(dᵢ)based on the patient information (Dₚ) for each dose reduction factor (dᵢ) and/or each CT protocol (Pᵢ), wherein the preview image (Im(dᵢ)) is a synthetic medical image simulating a medical image of the examination area based on the CT protocol (Pᵢ) and the dose reduction factor (dᵢ);
- providing (500) the generated preview images (Im(dᵢ)) to a user ;
- receiving a user selection of a generated preview image (Im(dᵢ) ) ;
- providing (600) the dose reduced CT protocol (Pᵢ) for the follow-up study, wherein the dose reduced CT protocol (Pᵢ) is the CT protocol (Pᵢ) related to the selected preview image (Im(dᵢ)).

## Description

The present invention relates to a computer-implemented method for providing a dose reduced CT protocol for a follow-up study of a patient. The present invention is settled in the technical field of medical imaging, and more specifically to methods and systems for reducing the radiation dose associated with computed tomography (CT) scans.

CT scanning is a widely used diagnostic tool in modern medicine, allowing healthcare providers to obtain detailed, three-dimensional images of internal organs and tissues. However, the high levels of ionizing radiation used in CT scans can pose risks to patients, particularly with repeated exposure. As a result, there is a growing need for technologies that can effectively reduce the radiation dose associated with CT imaging, without sacrificing image quality or diagnostic accuracy.

There are numerous strategies which deal with the technical and the scan parameters. Most strategies affect the image quality. Dose reduction in CT (computed tomography) refers to techniques or strategies that can be used to lower the amount of ionizing radiation that patients receive during a CT scan. Some of the parameters that can be adjusted to achieve dose reduction in CT include:
- mA (milliamperage): This parameter controls the amount of current flowing through the X-ray tube during the scan. Lowering the mA setting can reduce radiation dose, but it may also lead to increased noise in the image.
- kVp (kilovoltage peak): This parameter controls the energy of the X-ray beam. Lowering the kVp setting can reduce radiation dose, but it may also lead to increased image noise and reduced contrast.
- Pitch: The pitch is a measure of how fast the CT table moves relative to the speed of the X-ray beam. Increasing the pitch can reduce radiation dose, but it may also result in reduced image resolution.
- Scan length: Reducing the scan length to include only the necessary anatomical region can reduce radiation dose.
- Reconstruction algorithms: Using iterative reconstruction algorithms can help to reduce image noise and improve image quality, allowing for lower radiation dose.
- Shielding: Using lead shielding around sensitive areas of the body (e.g., the thyroid gland, breasts, and gonads) can reduce radiation dose.
- Automatic exposure control (AEC): AEC systems can automatically adjust the mA and kVp settings based on the size and shape of the patient's body, which can help to optimize radiation dose while maintaining image quality.

It's important to note that the specific parameters used for dose reduction in CT may vary depending on the patient's age, size, and clinical indication for the scan. Reducing the dose has a direct impact on the radiologist's ability to identify the imaging findings and reduce comfort with reviewing the images.

The present invention provides a novel approach for providing a dose reduced CT protocol for a follow-up scan of a patient, which may help to minimize the potential risks associated with medical imaging while still providing the valuable diagnostic information that physicians rely on.

Independent of the grammatical term usage, individuals with male, female or other gender identities are included with the term.

The method and device disclosed herein are interchangeable in that the features of the method can be applied to the device, and the features of the device can be applied to the method. Specifically, the method and device incorporate similar principles of operation, allowing for seamless integration between the two. This interchangeability enhances the overall functionality and versatility of the technology, providing a wide range of potential applications in various fields. Therefore, the disclosed method and device can be used individually or in combination with each other, depending on the specific needs of the user, and provide an innovative solution to the problem at hand.

The invention relates to a computer-implemented method for providing a dose reduced CT protocol for a follow-up study of a patient, comprising:
- receiving patient information of a primary study including at least one primary study, at least one primary CT protocol and/or at least one primary medical image of an examination area;
- receiving a follow-up information for the patient comprising at least one initial follow-up study and/or at least one initial follow-up CT protocol;
- receiving a plurality of dose reduction factors dᵢ, wherein the dose reduction factors dᵢ describe a reduction of a dose applied to a patient in a follow-up study with a CT protocol Pᵢ compared to a dosed applied to a patient in a follow-up study with the primary CT protocol;
- generating based on the patient information for each dose reduction factor dᵢ and/or each CT protocol Pᵢ at least one preview image Im(dᵢ), wherein the preview image Im(dᵢ) is a synthetic medical image simulating a medical image of the examination area acquired with the CT protocol Pᵢ and the dose reduction factor dᵢ;
- providing the generated preview images Im(dᵢ) to an user for selecting one of the generated preview images as an acceptable dose reduced image;
- receiving a user selection of a generated preview image Im(dᵢ) as the acceptable dose reduced image;
- providing the dose reduced CT protocol for the follow-up study, wherein the dose reduced CT protocol is the CT protocol Pᵢ related to the acceptable dose reduced image.

A computer-implemented method, also known as a software-implemented method or algorithm, is a process or set of instructions that is executed by a computer system or processor using software. The method is designed to solve a particular problem or accomplish a specific task, and typically involves one or more computer programs or applications.

The computer-implemented method according to the invention is designed to provide, determine and/or propose a CT protocol for a follow-up study of patient, wherein the CT protocol has a reduced dose and/or is optimized according to the dose, wherein optimized is for example a minimization of a dose applied in the CT scan of the follow-up study by preferably having a maximized or acceptable image quality. The instructions of the computer-implemented method are written in a programming language and are executed by the computer system, device or processor to perform the desired operations.

A CT (computed tomography) protocol refers to a set of guidelines and procedures used to perform a CT scan on a patient. The protocol defines the technical parameters of the scan, including the type of CT scanner to be used, the radiation dose, and/or the scanning parameters such as the slice thickness, scan speed, and/or contrast media injection protocols. The CT protocol may also include instructions on patient preparation, positioning, and breath-holding techniques to ensure the best possible image quality.

The CT protocol determines the diagnostic quality of the images produced by the CT scanner and can have a significant impact on patient outcomes. Standardized CT protocols help to ensure consistency and reproducibility in imaging, which can improve the accuracy of diagnoses and reduce the need for repeat scans. CT protocols are developed by radiologists and other medical professionals who specialize in CT imaging and are based on evidence-based best practices and safety guidelines.

In medical imaging, a primary study is a diagnostic imaging exam that is preferably performed to initially evaluate a patient's medical condition. Examples of primary studies include X-rays, computed tomography (CT) scans, magnetic resonance imaging (MRI), and ultrasound. These imaging exams are used to detect abnormalities, diagnose diseases, or evaluate the progression of a known medical condition. A primary study is especially used for planning a treatment of a patient, e.g., for planning a cancer treatment, radiation therapy, ablation therapy and/or chemotherapy.

A follow-up study, on the other hand, is a subsequent imaging exam that is performed to monitor the progression or treatment of a known medical condition. Follow-up studies are often performed after a primary study has identified a medical condition, to monitor the effectiveness of treatment or to detect any changes or progression of the condition. Follow-up studies are typically performed at regular intervals to track changes in the patient's medical condition and to evaluate the effectiveness of treatment.

In both primary and follow-up studies, medical imaging is used to provide physicians and other healthcare professionals with important diagnostic information that is used to make decisions about patient care. By providing detailed images of the internal structures of the body, medical imaging can help to diagnose diseases, monitor treatment, and guide surgical procedures, among other things.

A primary study and/or a follow-up study is based on an imaging protocol, here based on a CT protocol. In other words, the primary study and/or the follow-up studies is configured to acquire medical images (CT images) by usage of the imaging protocol and/or CT protocol. The phrases study and protocol can often be used synonymous.

In CT based imaging diagnosis, there are several procedures and/or studies, wherein the same scan protocol can be used for performing the primary and follow-up study. Examples for primary and follow-up studies for different anatomies are for example:

| Primary Study / Primary CT protocol | Follow-up Studies / Follow-up CT protocol |
|---|---|
| HeadNeuro.Adult | Follow-up of Intracranial Hemorrhage |
| | Follow-up of Hydrocephalus |
| | Follow-up of Post-surgical cases |
| | |
| AbdomenRoutine.Adult | Follow-up of Renal or ureteric stones |
| | Follow-up of Trauma cases |
| | Follow-up of chronic diseases |
| | Follow-up of vascular diseases |
| | |
| LungCARE.Adult | Follow-up of lung nodules. |
| | Follow-up of Pneumothorax / Hemothorax. |
| | Follow-up of pneumonia |
| | |
| Extremities | Follow-up of fractures |
| | Follow-up of known bone tumors |

Receiving patient information can be configured as collecting and/or obtaining the patient information. The patient information can be provided by a storage, e.g. a PACS or cloud. The patient information is preferably received as data and/or a data set. The patient information is related and/or specific for the patient. The patient information preferably comprises a primary study, e.g., a name of the primary study and/or a description of the primary study. The patient information preferably comprises a primary CT protocol, e.g., a name of the primary CT protocol, a description, settings and/or parameter of the primary CT protocol. The primary CT protocol is the CT protocol used for acquiring primary medical images and/or for performing the primary study. The patient information preferably comprises primary medical images, e.g., 2D or 3D medical images, especially CT images. The primary medical images are acquired in the primary study. The examination area comprises an anatomical area, anatomical structure and/or organ of the patient. The examination can comprise or describe a 2D or 3D section of body and/or the patient.

Receiving follow-up information can be configured as collecting and/or obtaining the follow-up information. The follow-up information can be provided by a storage, e.g. a PACS or cloud. Preferably, the follow-up information is provided by a user, e.g., as a user input. For example, a user chooses, plans, or selects a follow-up study and/or provides it as the follow-up information. The follow-up information is preferably received as data and/or a data set. The follow-up information is related and/or specific for the patient. The follow-up information preferably comprises at least one initial follow-up study and/or at least one initial follow-up CT protocol. The patient information preferably comprises a initial follow-up study. The initial follow-up study is for example a follow-up study chosen by a user, especially based on the primary study, the primary medical images and/or a diagnosis. Preferably, the initial follow-up study is a study recommended by internal, external or general guidelines. The initial follow-up study comprises for example a study name or study description. The initial follow-up CT protocol comprises or describes a description, settings and/or parameter of the CT protocol initially planned for the follow-up study.

Receiving a plurality of dose reduction factors dᵢ can be configured as collecting and/or obtaining the dose reduction factors dᵢ and/or a number and/or range of dose reduction factors dᵢ. The dose reduction factors dᵢ can be provided by a storage, e.g., a PACS or cloud. Preferably, the dose reduction factors dᵢ, the number and/or range of dose reduction factors dᵢ is provided by a user, e.g., as a user input. For example, a user chose, provides, or sets dose reduction factors di, e.g., by alphanumeric input. Alternatively, or additionally, the user can provide or set a number of dose reduction factors dᵢ and/or a range from where the number of dose reduction factors dᵢ can be chosen or provided. The dose reduction factors dᵢ describe a reduction of a dose applied to a patient in a follow-up study with a CT protocol compared to a dosed applied to a patient in a follow-up study with the primary CT protocol. The dose reduction factor dᵢ is especially a number between 0 and 1, preferably a percentage. The dose reduction factor dᵢ is for example the ratio between an initial dose Di and a follow-up dose Df, wherein the initial dose Di is the radiation dose applied to the patient in the primary study or by acquiring the primary medial images based on the primary CT protocol. The initial dose Di can also be the radiation dose applied to the patient in a follow-up study, especially based on the initial follow-up CT protocol. For example a user provides as a user input as a number N of dose reduction factors dᵢ the number N=5 and choses a lower limit 0.6 and as upper limit 0.9 for a dose reduction interval X=[0.6;0.9], the method provides 5 dose reduction factors dᵢ form the interval X, preferably choosing at dose reduction factors dᵢ equidistant. The plurality of dose reduction factors dᵢ comprises for i=1, 2, ..., N N dose reduction factors, e.g., d₁, d₂, ..., d_{N}.

The step generating at least one preview image Im(dᵢ) can be based on a machine learning algorithm, especially based on deep learning and/or neural networks. The step comprises preferably, generating, providing and/or simulating for each received dose reduction factor dᵢ a preview image Im(dᵢ). Especially, for each dose reduction factor dᵢ and each CT protocol Pn a preview image Im(dᵢ, Pn) is created, wherein the CT protocol Pn is the initial follow-up CT protocol and/or a CT protocol usable for the follow-up study. The preview image Im(dᵢ) is or appears like a medical image, especially a CT image or slice of a CT image. The preview image Im(dᵢ) comprises or shows the examination area of the patient. The preview image Im(dᵢ) is especially based on the primary medical images. The preview image is or appears like a CT image acquired with c CT protocol, especially CT protocols Pn, and a dose reduction factor dᵢ. The preview image Im(dᵢ) simulates in other words a medical image, which can be acquired in the follow-up study using the CT protocol Pn and a dose reduction factor dᵢ. The preview images Im(dᵢ, Pn) can also be understood as Im(dᵢ, Pn) and/or Im(dᵢ, Pn, K) and/or used synonymously.

The generated preview images Im(dᵢ), Im(dᵢ, Pn) and/or Im(dᵢ, Pn, K) are provided to an user for selecting one of the generated preview images as an acceptable dose reduced image. The preview images Im(dᵢ) are for example provided via an user interface, e.g., by presenting and/or displaying the preview images Im(dᵢ). The user can select or chose one of the provided preview images Im(dᵢ), e.g., by interacting with the user interface. The selected or chosen preview image Im(dᵢ) is named and/or provided as acceptable dose reduced image. Acceptable dose reduces means for example, that this preview image is according to the user sufficient for usage in the follow-up study, e.g., all necessary details are present.

The user selection, especially the acceptable dose reduced image and/or the related follow-up study and/or CT protocol P, is received, e.g., based on a user input and/or a storage medium. The CT protocol Pn and/or follow-up study related to acceptable medical image are provided, especially provided as the dose reduced CT protocol for the follow-up study. Preferably, a name, description, parameters and/or settings of the CT protocol related to the acceptable medical image is provided. The dose reduce CT protocol is especially a dose optimized CT protocol. The dose reduced CT protocol can be provided as ready to use CT protocol and/or as machine readable data.

The invention is based on the idea of using prior knowledge, especially a prior study, prior CT protocol, and/or prior medical images to generate and provide preview images for different dose reduction factors dᵢ. Based on the preview images Im(dᵢ), a user can decide which one is acceptable and/or enough for the purpose of the follow-up study. For example, the user can choose a preview image Im(dᵢ) with a large dose reduction factor dᵢ but having enough details to be used in the follow-up study.

According to an optional embodiment of the invention, the step of generating at least one preview image Im(dᵢ) comprises providing a number K of axial slices and/or generating, for each dose reduction factor dᵢ, K preview images Im(dᵢ, k), wherein k=1, 2, ..., K. Providing the number K can be configured or can comprise providing not just a number K of slices but providing or defining axial slices k themselves. The number K can be an integer between one and 20, especially a number between two and 15 and preferably a number between five and 10. The number K defines the number of preview images Im(dᵢ) that are generated for each dose reduction factor and/or each protocol. For example, for a number K equal to five axial slices k₁, k₂, k₃, k₄, k₅, five preview images Im(dᵢ, k₁), Im(dᵢ, k₂), Im(dᵢ, k₃), Im(dᵢ, k₄), Im(dᵢ, k₅) will be generated for a combination of dose reduction factor dᵢ and/or protocol Pn. This embodiment is based on the idea of providing to a user, for each dose reduction factor dᵢ and/or each protocol Pn, a plurality of preview images Im(dᵢ, k) each presenting a different axial slice to choose and/or set the acceptable medical image. Alternatively and/or additionally sagittal slices and/or coronal slices can be used. Especially, the features for the axial slices can be read and used as sagittal and/or coronal slices.

In a CT scan, an axial slice refers to a 2-dimensional image that is obtained by taking a cross-sectional view of the body or examination area in a plane that is parallel to the ground or horizontal plane. The axial plane is one of the three anatomical planes, along with the sagittal plane and the coronal plane, which are commonly used in medical imaging to visualize internal structures of the body. CT scans consist of a series of axial slices, which can be stacked together to create a 3-dimensional image of the body. Axial slices are particularly useful in identifying abnormalities in structures that lie in the horizontal plane, such as the brain or abdomen.

According to a preferred embodiment of the invention, the step of generating the preview images Im(dᵢ), especially Im(dᵢ, k) and/or Im(dᵢ, Pn, k), is based on and/or comprises a trained function. For example, generating the preview images Im(dᵢ) is based on applying the trained function to input data, wherein the input data comprises the received dose reduction factors dᵢ, the patient information and/or the follow-up information. The trained function is trained, especially based on training data, and/or is configured to generate output data based on input data, wherein the output data comprises the preview images Im(dᵢ), Im(dᵢ, k), and/or Im(dᵢ, Pn, k). The trained function is configured and/or trained to generate a simulated medical image, especially a simulated CT image, showing the examination area of the patient as acquired based on the CT protocol and the dose reduction factor dᵢ. The trained function is a trained function according to and/or based on matching learning, especially based on deep learning. For example, the trained function is configured and/or based on the neural network, especially the neuronal network. By applying the trained function to the input data, at least one preview medical image Im(dᵢ, k₁) is generated as output data. The generated output data and/or comprised preview image is provided to the user for selecting the acceptable medical image.

Preferably, the trained function is based on and/or comprises a generic adversarial network. Alternatively and/or additionally, the trained function is based on and/or comprises an unsupervised trained function, for example, an unsupervised image-to-image translation network. The image-to-image translation network is a network, especially the neuronal network, configured to generate an image, especially a medical image, as output data based on input data, wherein the input data comprises and/or is configured as an image, especially a medical image. The trained function is preferably based and/or configured as described in "An Introduction to Image Synthesis with Generative Adversarial Nets" (He Huang, Philip S. Yu and Changhu Wang, https://arxiv.org/abs/1803.04469) and/or "Unsupervised Image-to-Image Translation Networks" (Ming-Yu Liu, Thomas Breuel, Jan Kautz, https://arxiv.org/abs/1703.00848).

GAN stands for Generative Adversarial Networks. It is a type of deep learning model that is used for generating new, realistic data from a given dataset. GANs consist of two neural networks: a generator and a discriminator. The generator's job is to create new data that is similar to the original dataset. It takes random noise as input and generates new data that is fed into the discriminator. The discriminator's job is to distinguish between the generated data and the original dataset. It is trained on the original dataset to recognize the characteristics of the real data. During training, the generator and discriminator networks compete against each other in a game-like setting. The generator tries to create data that is similar enough to the original dataset to fool the discriminator, while the discriminator tries to accurately distinguish between the generated data and the real data.

Over time, the generator becomes better at creating data that is realistic enough to fool the discriminator. As the training progresses, the generator learns to generate data that is increasingly similar to the original dataset.

An unsupervised image-to-image translation network is a type of machine learning model that is trained to transform images from one domain to another without the need for paired training data. This means that unlike supervised image-to-image translation networks, which require images in the source and target domains to be paired with each other during training, unsupervised models can learn to translate between domains using only unpaired data.

Unsupervised image-to-image translation networks typically use a type of deep learning architecture known as a generative adversarial network (GAN). In a GAN, two neural networks are trained simultaneously: a generator network, which learns to create images that mimic the target domain, and a discriminator network, which learns to distinguish between real and generated images. The generator network is trained to fool the discriminator by producing images that are indistinguishable from real images, while the discriminator network is trained to become increasingly better at telling real and generated images apart. During training, the generator and discriminator networks are updated iteratively to improve the quality of the generated images. Once training is complete, the generator network can be used to translate images from the source domain to the target domain by inputting a source image and using the generator to produce a translated image. The translated image is then outputted by the network as the final result. Unsupervised image-to-image translation networks have been used in a variety of applications, such as style transfer, image colorization, and medical image segmentation.

In the context of image processing and machine learning, an image domain refers to a specific type or category of images that share common characteristics. For example, medical images such as CT scans or MRI images belong to the medical image domain, while satellite images or street view images belong to the outdoor image domain. Understanding the image domain is important in image processing tasks such as image segmentation, object detection, and image-to-image translation, where the algorithm needs to learn the specific characteristics and patterns of the images in the domain in order to perform accurate and reliable analysis or transformation.

According to an optional embodiment of the invention, the trained function is configured and/or trained to transfer and/or map an input image to an output image. The input image is of a source domain, whereas the output image is of a target domain. The first domain corresponds to a medical image, especially a CT image, axial slice, acquired with the CT protocol PI and the dose reduction factor equal to one. Especially, the first domain corresponds to a primary medical image, which is acquired based on the primary study and/or primary CT protocol. In other words, the target domain is based on medical images comprised of patient information data. The output domain or target domain corresponds to a medical image acquired with the CT protocol and a dose reduction factor dᵢ. For example, the initial follow-up CT protocol and the dose reduction factor dᵢ. The transfer and/or mapping of the images is, in other words, a mapping and/or transfer of medical images to medical images of a different domain, e.g., a different dose reduction factor and/or different CT protocol.

Preferably, the trained function is trained for and/or dependent on the initial follow-up study and/or the initial follow-up CT protocol. For example, the method can comprise a plurality of trained functions, especially trained functions for each different initial follow-up study and/or initial follow-up CT protocol. For instance, one trained function is trained and/or dependent on medical images comprising an abdomen as the examination area, and a different one is trained for and/or dependent on a study and/or medical images comprising the whole body of the patient as the examination area.

According to an embodiment of the invention, the step providing the dose reduction factor di may include determining the dose reduction factors di based on a time interval. Therefore, a time interval between the date and/or timestamp of acquiring the primary study and/or primary medical images and acquiring the follow-up study and/or acquiring the medical images is determined. In other words, the time interval is the amount of time between the primary study and the follow-up study. The dose reduction factors di are, for example, determined based on the number of dose reduction factors and the time interval. For instance, for the same number of dose reduction factors, e.g., five, different five dose reduction factors di are determined for different intervals. For example, for a small-time interval between the primary and the follow-up study, larger dose reduction factors di are data mined and/or provided. Whereas for a larger time interval between the primary study and the follow-up study, smaller dose di reduction factors with related dose reduction factors near and/or equal to one are provided. This is based on the idea that larger changes can be expected in the examination area for a larger time interval between the primary study and the follow-up study compared to a smaller time interval.

Preferably, the step providing the generated preview images comprises providing the preview images Im(di) together with the related dose reduction factor di. For example, for each dose reduction factor di, the related preview image and/or preview images, e.g., axial slices, are provided, especially as a dataset and/or table of information. Providing the preview images can also be configured as providing a table and/or list comprising the dose reduction factors and/or CT protocols together with the related medical images, especially the preview images.

Preferably, the step providing the generated preview images comprises providing the preview images Im(dᵢ) in a ranked order. The ranked order is especially a ranked order according to the dose reduction dᵢ for example in a decreasing or increasing manner. For example, the preview image Im(dᵢ) with the highest or lowest dose reduction factor dᵢ is provided first.

According to an optional embodiment, ranking of the preview images Im(dᵢ) is based on a user preference, especially by applying a second trained function to the generated preview images Im(dᵢ). The second trained function is trained to generate a ranking of preview images Im(dᵢ) according to a dose reduction factor dᵢ and or according to the user preference. For example, a user has choosing medical image Im(dᵢ) with an average dose reduction factor as acceptable medical image instead of one with the highest or lowest dose reduction factor, so that the next times the preview image with the related often choosing dose reduction factor is ranked higher.

The invention is also related to a computer program, especially executable by a computer, device, and or processor. The computer program is configured to execute and/or implement the method according to the invention by the computer program is executed and or running.

The invention is also related to a computer program product, especially to the data storage, wherein the computer program is stored and or safety on the storage medium.

The invention is also related to the device for providing the dose reduced CT protocol for a follow-up study of a patient. The device is especially configured and/or adapted for running and/or executing the computer implemented method according to the invention. The device is for example a smart device, e.g. a tablet, smart phone or computer. The computer the device comprises a data storage, and interface, and or processor. The interface is configured to receive the patient information, at least one dose reduction factor dᵢ and or the initial follow-up information The processor is configured to include, implement and/or execute the features of claim one. The processor is configured to generate based on the patient information for each dose reduction factor dᵢ and/or each CT protocol Pᵢ at least one preview image Im(dᵢ), wherein the preview image Im(dᵢ) is a synthetic medical image simulating a medical image of the examination area acquired with the CT protocol Pᵢ and the dose reduction factor dᵢ. The processor and/or the interface is configured to provide the generated preview images Im(dᵢ) to an user for selecting one of the generated preview images as an acceptable dose reduced image. The interface is configured to receive a user selection of a generated preview image Im(dᵢ) as the acceptable dose reduced image. The interface and/or processor is configured to provide the dose reduced CT protocol for the follow-up study, wherein the dose reduced CT protocol is the CT protocol Pᵢ related to the acceptable dose reduced image.

Further details, embodiments, and or advantages are comprised in the figures and its description. The figures show
- Figure 1: a flow-chard of a method for providing a dose reduced CT protocol;
- Figure 2: a blockview of a device for providing a dose reduced CT protocol;
- Figure 3: an overview of an data flow in a method for providing a dose reduced CT protocol;
- Figure 4: a schematic method for selecting a trained function for a method to provide a dose reduced CT protocol;
- Figure 5: a schematic flow-chard for a trained function to provide a dose reduced CT protocol.

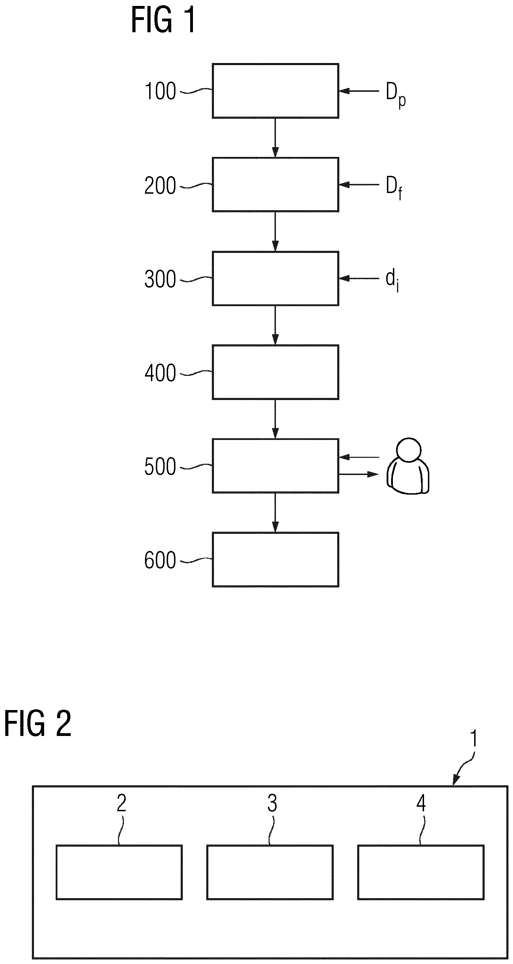

Figure 1 shows an example of a flowchart depicting an embodiment of the computer-implemented method for determining and/ or providing a dose-reduced CT protocol. The method can be implemented and/or executed by a device, especially by a computer, a cloud computing environment, and/or a computer tomography device.

Step 100 refers to the first step of the computer-implemented method for determining and/or providing a dose-reduced CT protocol. In this step, patient information Dₚ is provided, which is essential for the rest of the process to proceed. The patient information Dₚ can be provided through various means such as an interface, a storage device, or directly by the user.

The patient information Dₚ is related and/or acquired in a primary study, which involves medical imaging, particularly computed tomography (CT) of the patient. The primary study could be a previous imaging session that has already been conducted.

The patient information Dₚ includes a primary CT protocol and/or information about the primary study. The primary CT protocol refers to the protocol, parameterization, and/or settings of the CT protocol when the patient is medically imaged in the primary study. It may consist of a name of the CT protocol and/or parameters and/or settings for scanning a patient with the CT device. The patient information Dₚ may also include primary medical images, especially primary CT images, which were acquired of the patient, especially in the primary study and/or using the primary CT protocol.

Step 200 refers to the receiving of follow-up information D_{f}. The follow-up information D_{f} can be received through different means such as an interface or by a user, device, storage, and/or human-machine-interface. This information D_{f} is intended for a planned follow-up study of the patient, which is especially based on the results and/or findings of the primary study.

The follow-up information can also include the initial follow-up study and/or initial follow-up CT protocol. The word 'initial' in this context means that the follow-up study or CT protocol is suggested based on guidelines, medical practice, and/or by a user, for example, choosing a follow-up study out of possible follow-up studies.

The initial follow-up CT protocol can either be the same as the primary CT protocol or it can be a different CT protocol. In either case, it is preferably a more specific and/or specialized CT protocol, tailored to the needs of the patient based on the results and/or findings of the primary study.

Step 300 is related to a providing of dose reduction factors dᵢ. The user can preferably set and/or choose a plurality of dose reduction factors dᵢ, for example, 10 dose reduction factors d₁, d₂, ..., d₁₀. These factors dᵢ describe the amount of radiation dose applied to the patient when acquiring CT images with the rate-related CT protocol, settings and/or parameters compared to acquiring CT images with the primary CT protocol, settings and/or parameters.

The dose reduction factors dᵢ can also be received by the system as preset and/or fixed dose reduction factors dᵢ. The factors dᵢ could depend on the time interval between the primary study and the follow-up study. For instance, for a larger time interval between the primary study and the follow-up study, a larger dose reduction factor is received. This means that almost the same radiation dose as in the primary study is applied to the patient, ensuring accurate and consistent results.

The provision of dose reduction factors dᵢ is essential in reducing the amount of radiation exposure to the patient during the follow-up study. By reducing the amount of radiation dose, the patient's risk of developing radiation-induced complications or cancer is minimized. The dose reduction factors dᵢ can be adjusted according to the patient's individual needs, ensuring a personalized and safe approach to the follow-up study.

This method involves generating preview images Im(dᵢ) that are based on applying a trained function to input data, which includes patient information Dₚ, follow-up information D_{f}, and dose reduction factors dᵢ. Especially, for different CT protocols Pᵢ, different settings s and/or parameter p of the CT protocols Pᵢ(p), Pᵢ(s), Pᵢ(p,s) different preview images Im(dᵢ, Pᵢ), Im(dᵢ, Pᵢ(s)) Im(dᵢ, Pᵢ(s)) and/or Im(dᵢ, Pᵢ(p,s)) are generated.

The trained function can be obtained from a variety of sources, including a storage device, cloud service, or another device. Once the trained function is obtained, it can be used to generate a plurality of preview images Im(dᵢ) in step 400. For each dose reduction factor dᵢ, at least one preview image is generated, and the generated preview images are provided to the user in step 500.

The user can then choose an acceptable preview image Im(dᵢ) that shows enough details and/or has good enough quality while also having a high dose reduction factor. This acceptable preview image is used to determine the dose reduced CT protocol in step 600, which is the CT protocol that can be used to acquire CT images similar to the acceptable preview image.

By using this method, healthcare professionals can optimize the CT imaging process to achieve the desired level of image quality while reducing the patient's exposure to radiation. Additionally, by providing the preview images to the user, they can have a more active role in the decision-making process and have a better understanding of the imaging procedure.

Figure 2 is describing a device 1 that is designed to provide a dose-reduced protocol for a CT scan. The device 1 consists of several components, including an interface 2, a processor 3, and a storage system 4.

The interface 2 is a human-machine interface, which means that it is designed to allow communication between the device 1 and a human operator. It is configured to implement steps 100, 200, and 300 of the method according to Figure 1. This means that the interface 2 is responsible for carrying out these specific steps of the method.

The processor 3 is another component of the device 1, and it is configured to implement and/or execute step 400 according to the method of Figure 1. This step is likely to involve some computation or data processing, which the processor is capable of carrying out.

Finally, the storage 4 of the device 1 is configured to provide the trained function, the dose reduction factors, and/or receive and/or save the provided dose-reduced CT protocol. Additionally, it can receive the provided dose-reduced CT protocol and save it for future use.

Overall, the statement is describing a sophisticated device 1 that is capable of reducing the dose of radiation used in a CT scan, while still maintaining image quality. This has the potential to significantly reduce the risk of radiation exposure for patients undergoing CT scans.

In addition to the components mentioned in the statement, the device 1 may also include other hardware and software components that are specifically designed for the purpose of providing a dose-reduced protocol for CT scans. For example, it may include sensors that can measure the radiation dose received by the patient in real-time, allowing the device to make adjustments to the dose based on the patient's individual needs.

The device 1 may also be equipped with advanced algorithms that can analyze the data generated by the CT scan and make adjustments to the dose based on factors such as the patient's age, weight, and medical history. This can help ensure that the patient receives the optimal dose of radiation for their specific needs, while minimizing the risk of unnecessary exposure.

By reducing the dose of radiation used in CT scans, the device 1 described in the statement can help mitigate the potential risks associated with radiation exposure, including an increased risk of cancer and other health problems. At the same time, it can help improve the accuracy and quality of CT scans, enabling healthcare providers to make more informed diagnoses and treatment decisions.

Overall, the device 1 described in the statement is an important technological advancement in the field of medical imaging, offering significant benefits for both patients and healthcare providers

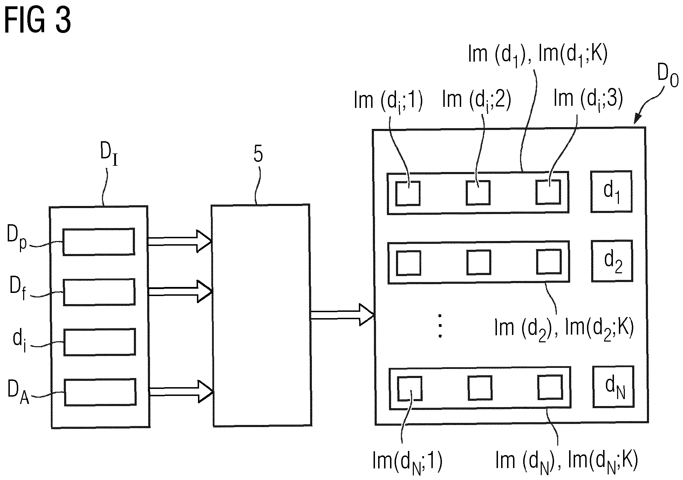

Figure 3 shows an example of a dataflow in the method for providing a dose reduced CT protocol. The method can be based and or configured as shown in figure 1. To a trained function, which is configured as a GAN 5, input data D_{I} are provided. The input data D_{I} comprise the patient information Dₚ, the follow-up information D_{f} and dose reduction factors dᵢ. Furthermore, the input data D_{I} can comprise additional data D_{A}, for example medical images acquired prior to the follow-up study. The medical images provided additional information D_{A} can be acquired using different modalities, for example, MRI images or pet images.

The trained function is configured to generate based on the input data D_{I} output data D_{O}. The output data D_{O} comprise preview images Im(dᵢ). The output data D_{O}, especially the preview images Im(dᵢ) are provided and or shown to a user, for example on a monitor. The preview images Im(dᵢ) are provided together with the related dose dᵢ reduction factor, so that the user can choose the acceptable preview image based on the image itself and the dose reduction factor dᵢ. Especially, for each dose reduction factor dᵢ K preview images Im(dᵢ, k) are generated and provided, wherein k=1, 2, ..., K defines different axial slices through the examination area.

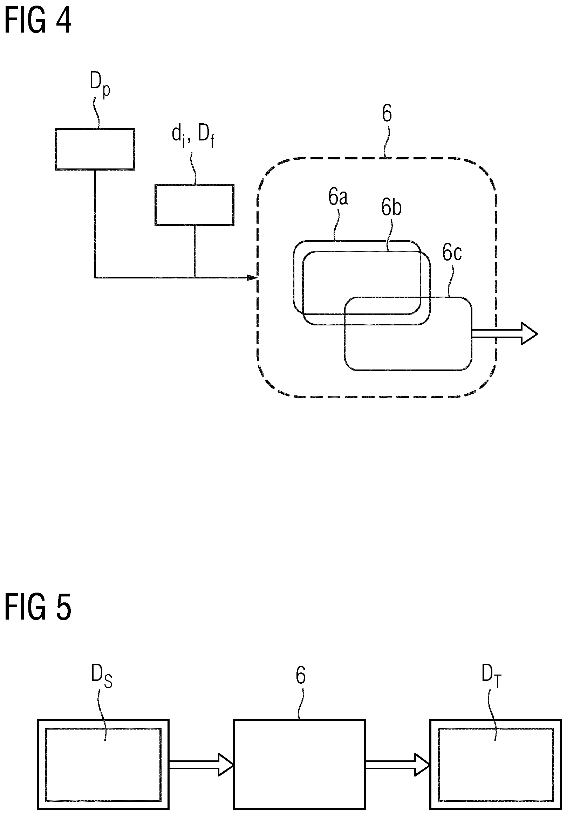

Figure 4 describes the data flow in a method for providing a dose-reduced CT protocol. The method is based on and configured as shown in Figure 1. A trained function, configured as a GAN 5, receives input data D_{I} that includes patient information Dₚ, follow-up information D_{f}, and at least one dose reduction factor dᵢ. The input data D_{I} may also include additional data D_{A}, such as medical images acquired using different modalities.

The trained function generates output D_{O} data that includes preview images Im(dᵢ), which are shown to the user on a human-machine interface. The user can choose an acceptable preview image based on the image quality and the associated dose reduction factor dᵢ. The trained function is configured as a GAN 5 and is trained to map a CT image from a source domain D_{S} to a CT image of a target domain D_{T}. The trained function is an unsupervised trained function.

Figure 4 shows that the computer implemented method and/or device 1 uses different trained functions, e.g., GANs 6, 6a, 6b, 6c depending on the primary CT protocol and/or initial follow-up CT protocol of a patient.

The choice of which trained function to use is based on considering the different target domains D_{T} and/or different source domains D_{S} involved. Target domain D_{T} refers to the domain to which the trained function maps the input data, while source domain D_{S} refers to the domain from which the input data comes.

To choose or receive the appropriate trained function, patient information D such as the primary study or primary CT protocol is taken into account. Follow-up information D_{f}, such as the initial follow-up study or initial follow-up CT protocol, is also considered. By choosing the appropriate trained function based on this information, the computer implemented method and/or device is better able to provide a dose-reduced CT protocol that is tailored to the specific needs of each patient.

Figure 5 shows, that the trained function, especially adapted and/or configured as a GAN 5, is trained and or configured to map a CT image comprised by the input data D_{I} to a CT image for the output data D_{O}. The trained function is configured to map the medical CT images of an input and or source domain to our medic CT images of a target domain. The trained function is especially unsupervised trained function.

A further example of an embodiment of the invention is described here.

Herewith an AI based dose optimization tool is proposed for follow-up scans. Henceforth, we refer this as FollowupDoseOptimizer. The dose reduction factor, referred as ,d' is the ratio of the dose in follow-up study / primary study in %. This tool will be used by the radiographer who configures the scan parameters (e.g. dose) for a given procedure, prior to the scan process.

If the radiographer is experienced/ trained in analyzing the images, he would decide on the optimal dose value. Else, he could take an approval from the radiologist who would analyze the image (as part of the diagnosis).

The workflow steps are as follows:
List of notations, configurable values:
Tp : Time of primary study
Tf : Time of follow-up study
C1 : interval between primary and follow-up studies (<=2 weeks)
C2 : interval between primary and follow-up studies (> 2 weeks)
N : number of options of ,d' (e.g., N can be a small number, say 5).
K : number of axial slices (e.g., K could be 3 or 5).
Δ : the base offset between two slices of the generated image.
L : Number of studies (learning period) for the ranking step.

### Steps:

```
-------------------------------------------------------------
 -----------------------------------------------------
 User selects patient / loads data for primary study
 Creates a follow-up study
 User selects scan protocol
 If (primary scan protocol is same as the follow-up protocol)
 then
 {
 User selects dose parameter customization options
 FollowupDoseOptimizer computes the interval between primary,
 follow-up studies and maps into one of the two categories
 (C1, C2)
 For each category (C1 or C2), there are defined set of dose
 reduction factors {d[i] : i = 1 .. N}
 For each dose factor d[i], K image slices are simulated (Re-
 fer Section 3.2)
```

ImS [] [1.. K] corresponds to image slices with numbers { Rk : k = 1 to K}. Rk is a random number with integral axial shifts of Δ.

It displays list of N "% dose reduction factors" and the corresponding image sample sets <d[i], ImS[i][1..K]>. (i = 1..N).

User selects the acceptable dose reduction factor.

User performs other configurations/ selections for follow-up study. } Else User configures the follow-up protocol in the conventional process.

### User proceeds with the follow-up scan.

If the interval between the studies is less (C1 category), higher level of dose reduction is proposed. Else, lesser dose reduction is proposed. And the ranges of 'd' would be selected accordingly.

For the intitial L studies (after deployment), FollowupDoseOptimizer displays <d[i], Im[i]>, with ascending order of d[i]. After that, it ranks the combinations depending on the user preferred 'optimal dose'.

### Section 3.2 Image Simulation

The proposed design uses a Generative Adversarial Network (GAN) for generating /simulating the images. **(Refer [1]).** FollowupDoseOptimizer **has a GAN model for each type of primary study and the dose % factor, d. For example, if there are four primary studies (as in the table below) and five possible dose factors d, then there would be 20 models.**

We propose to use UN-supervised Image-to-image Translation (UNIT) network for this purpose.

The details of this specific GAN model are provided in [2]. The implementation of UNIT network is given in [3].

UNIT network is a GAN based model that can be used for image-to-image mapping where the input image belongs to a source domain Ds and output belong to target domain Dt.

For this use case, Ds corresponds to the domain with 100% dose (with best image quality)
Dt corresponds to the target domain (image with reduced dose); since we have multiple dose options and primary studies, there would be corresponding target domains, say Dt [p][d], where p, d are indices for primary study and dose factor.

### Training phase:

During the training phase, the network parameters would be optimized based on sample images from Ds , Dt domains.

The UNIT network is unsupervised as it does not need training images in [Ds , Dt ] as corresponding pairs. This makes the data preparation step simple, as we don't need image acquisition of the same anatomy at different dose values.

### Refer [3] for further details on training.

### Generation phase:

During generation phase, the network takes the image (slices) of the primary study and generates equivalents in target domains. A random index generator picks up axial slices from the primary image set and feeds the UNIT nets.

Since we have separate UNIT nets for each target domain Dt [p][d], the image generation quality is expected to be high and specific for the anatomy.

## Claims

1. A computer-implemented method for providing a dose reduced CT protocol for a follow-up study of a patient, comprising:
- receiving (100) patient information (Dₚ) of a primary study, including at least one primary CT protocol and/or at least one primary medical image of an examination area;
- receiving (200) a follow-up information (D_{f}) comprising at least one initial follow-up CT protocol;
- receiving (300) a plurality of dose reduction factors (dᵢ), wherein the dose reduction factors (dᵢ) define a dose reduction for a follow-up study with a CT protocol (Pᵢ) compared to a follow-up study with the primary CT protocol;
- generating (400) at least one preview image Im(dᵢ)based on the patient information (Dₚ) for each dose reduction factor (dᵢ) and/or each CT protocol (Pᵢ), wherein the preview image (Im(dᵢ)) is a synthetic medical image simulating a medical image of the examination area based on the CT protocol (Pᵢ) and the dose reduction factor (dᵢ);
- providing (500) the generated preview images (Im(dᵢ)) to a user ;
- receiving a user selection of a generated preview image (Im(dᵢ));
- providing (600) the dose reduced CT protocol (Pᵢ) for the follow-up study, wherein the dose reduced CT protocol (Pᵢ) is the CT protocol (Pᵢ) related to the selected preview image (Im(dᵢ)).

2. Computer-implemented method according to claim 1, wherein the step generating (400) at least one preview image (Im(dᵢ)) comprises:
- providing a number K of axial slices;
- generating for each dose reduction factor (dᵢ) K preview images (Im(dᵢ, k)), wherein k=1,...,K is a slice number, wherein the preview image (Im(dᵢ, k)) is a synthetic medical image (Im(dᵢ)) simulating a medical image of the axial slice k of the examination area acquired with the CT protocol (Pᵢ) and the dose reduction factor di.

3. Computer-implemented method according to claim 1 or 2, wherein the step generating (400) at least one preview image (Im(dᵢ)) comprises:
- providing at least one trained function, wherein the trained function is configured to generate a preview image (Im(dᵢ)) when applied to patient information (Dₚ), the follow-up information (D_{f}), the CT protocols (Pᵢ) and/or a dose reduction factor (dᵢ);
- generating the preview image (Im(dᵢ)) based on applying the trained function to the patient information (Dₚ), the follow-up information (D_{f}), the CT protocols (Pᵢ) and/or the dose reduction factors (dᵢ).

4. Computer-implemented method according to claim 3, wherein the trained function is based on a GAN (5) and/or based on an unsupervised image-to image translation network.

5. Computer-implemented method according to claim 3 or 4, wherein the trained function configured to transfer and/or map an input image of a source domain (D_{S}) to an output image of a target domain (D_{T}), wherein the source domain (D_{S}) corresponds to a medical image acquired with the primary CT protocol and/or the CT protocol (Pᵢ) and a dose reduction factor dᵢ=1 and the target domain (D_{T}) corresponds to a medical image acquired with the CT protocol (Pᵢ) and a dose reduction factor (dᵢ) unequal to 1.

6. Computer-implemented method according to one of the claims 3 to 5, wherein the trained function is trained for and/or depends on the initial follow-up CT protocol.

7. Computer-implemented method according to one of the previous claims, wherein the step providing (300) the dose reduction factors (dᵢ) comprises:
- Determining a time interval between acquiring the primary study and the follow-up study;
- Determining dose reduction factors (dᵢ) based on the determined time interval, wherein the dose reduction factor (dᵢ) is correlated to the time interval.

8. Computer-implemented method according to one of the previous claims, wherein the step providing (500) the generated preview images (Im(dᵢ)) to the user comprises:
- providing the preview image (Im(dᵢ)) together with the related dose reduction factor (dᵢ).

9. Computer-implemented method according to one of the previous claims, wherein the step providing (500) the generated preview images (Im(dᵢ)) to the user comprises:
- providing the preview images (Im(dᵢ)) in a ranked order, wherein the ranked order is based on a ranking according to the dose reduction factors (dᵢ).

10. Computer-implemented method according to one of the previous claims, wherein the step providing (500) the generated preview images (Im(dᵢ)) to the user comprises:
- providing the preview images (Im(dᵢ)) in a ranked order, wherein the ranked order is based on a ranking according to a user preference.

11. Computer-implemented method according to claim 10, wherein the step providing the generated preview images (Im(dᵢ)) to the user comprises:
- ranking the preview images (Im(dᵢ)) based on a user preference by applying a second trained function to the generated preview images (Im(dᵢ)).

12. Computer-implemented method according to claim 11, wherein the step receiving a user selection comprises:
- training the second trained function based on the user selection and the generated preview images (Im(dᵢ)),
- providing the trained second trained function.

13. Computer program configured for execution and/or running on a computer, processor (3) and/or device (1), wherein the computer program is configured to process the method according to one of the previous claims when executed and/or running on the computer, processor (3) or device (1).

14. Machine-readable medium, wherein the computer program according to claim 13 and/or program code implementing the computer program according to claim 13 is comprised by, stored and/or saved on the medium.

15. Device for providing a dose reduced CT protocol for a follow-up study of a patient, comprising an interface (2) and a processor (3), wherein the interface (2) is configured to receive patient information (Dₚ) of a primary study, including at least one primary CT protocol and/or at least one primary medical image of an examination area; to receive a follow-up information (D_{f}) comprising at least one initial follow-up CT protocol; to receive a plurality of dose reduction factors (dᵢ), wherein the dose reduction factors (dᵢ) define a dose reduction for a follow-up study with a CT protocol (Pᵢ) compared to a follow-up study with the primary CT protocol; wherein the processor is configured to generate at least one preview image Im(dᵢ)based on the patient information (Dₚ) for each dose reduction factor (dᵢ) and/or each CT protocol (Pᵢ), wherein the preview image (Im(dᵢ)) is a synthetic medical image simulating a medical image of the examination area based on the CT protocol (Pᵢ) and the dose reduction factor (dᵢ); wherein the interface (2) is configured to provide the generated preview images (Im(dᵢ)) to a user, to receive a user selection of a generated preview image (Im(dᵢ)) and to provide the dose reduced CT protocol (Pᵢ) for the follow-up study, wherein the dose reduced CT protocol (Pᵢ) is the CT protocol (Pᵢ) related to the selected preview image (Im(dᵢ)).
